(19) Europäisches Patentamt
European Patent Office
Office européen des brevets

(11) Veröffentlichungsnummer: **0 334 118 B1**


(12) # EUROPÄISCHE PATENTSCHRIFT


(45) Veröffentlichungstag der Patentschrift: **11.11.92**

(51) Int. Cl.⁵: **C07C 29/136**, C07C 31/125, C07C 31/20

(21) Anmeldenummer: **89104240.0**

(22) Anmeldetag: **10.03.89**


(54) **Verfahren zur katalytischen Hydrierung von flüssigen Fettsäure-Triglyceriden zur gleichzeitigen Gewinnung von Fettalkoholen und C3-Diolen.**


(30) Priorität: **19.03.88 DE 3809270**

(43) Veröffentlichungstag der Anmeldung:
**27.09.89 Patentblatt 89/39**

(45) Bekanntmachung des Hinweises auf die Patenterteilung:
**11.11.92 Patentblatt 92/46**

(84) Benannte Vertragsstaaten:
**BE DE FR GB NL SE**

(56) Entgegenhaltungen:
**EP-A- 0 254 189**
**DE-A- 2 853 990**

**CHEMIE-TECHNIK, 4. Jahrgang, Nr. 12, 1975, Dr. Alfred Hüthig Verlag, Heidelberg; W. SWODENK, "Hydrierung in der Rieselphase-Verfahrenstechnik und Anwendungsmöglichkeiten", Seiten 439-441**



(73) Patentinhaber: **Henkel Kommanditgesellschaft auf Aktien**
**Postfach 1100 Henkelstrasse 67**
**W-4000 Düsseldorf 1(DE)**

(72) Erfinder: **Fleckenstein, Theo, Dr.**
**Pfitznerstrasse 9**
**W-4010 Hilden(DE)**
Erfinder: **Göbel, Gerd, Dr.**
**Lindenstrasse 10**
**W-4006 Erkrath(DE)**
Erfinder: **Carduck, Franz-Josef, Dr.**
**Landstrasse 18**
**W-5657 Haan(DE)**
Erfinder: **Bremus, Norbert**
**Turmstrasse 21**
**W-4019 Langenfeld(DE)**
Erfinder: **Eltner, Reinhard**
**Am Falder 57**
**W-4000 Düsseldorf 13(DE)**

(74) Vertreter: **Patentanwälte Meinke und Dabringhaus Dipl.-Ing. J. Meinke Dipl.-Ing. W. Dabringhaus**
**Westenhellweg 67**
**W-4600 Dortmund 1(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur katalytischen Hydrierung von flüssigen Fettsäure-Triglyceriden zur gleichzeitigen Gewinnung von Fettalkoholen und $C_3$-Diolen in Gegenwart von gasförmigem Wasserstoff und Hydrierkatalysatoren bei Drücken von 50 bis 300 bar und Temperaturen von 160 bis 250 ° C.

Ein derartiges Verfahren ist aus der deutschen Patentanmeldung DE 36 24 812 der Anmelderin bekannt. Dabei werden Katalysatoren auf Basis Kupferchromit eingesetzt,mit denen es möglich ist, unter vergleichsweise moderaten Reaktionsbedingungen mit hoher Aktivität und Selektivität die direkte Hydrierung von Triglyceriden zu Fettalkoholen so zu steuern, daß als wertvolles Nebenprodukt 1,2-Propandiol entsteht, das für die Herstellung von Alkyd- oder Polyesterharzen sowie für zahlreiche andere Anwendungen eingesetzt werden kann.

Da diese, wie auch andere Hydrierkatalysatoren, bei einer Erhöhung der Verweilzeit des Reaktionsgemischs unter Hydrierbedingungen den Abbau des Wertprodukts 1,2-Propandiol zu Folgeprodukten und den Abbau der Fettalkohole zu Paraffinen katalysieren, läßt sich die Hydrierreaktion nur auf der Stufe der gewünschten Produkte anhalten, wenn spezielle Verfahrensbedingungen eingehalten werden. Aus der DE 36 24 812 ist aber nicht bekannt, wie die katalytische Hydrierung von Triglyceriden großtechnisch verwirklicht werden kann.

Derartige katalytische Reaktionen werden vielfach in Festbettreaktoren durchgeführt, in denen die fluiden Phasen als Rieselphase in den Reaktor geleitet werden. Solche Reaktoren sind beispielsweise in Ullmanns Enzyklopädie der technischen Chemie, 4. Aufl., Bd. 3, S. 500 ff. beschrieben. Die Reaktionsführung in diesen Reaktoren erfolgt adiabatisch, d.h. bei den meist exothermen Reaktionen steigt die Temperatur längs der Katalysatorschüttung an. Da die Selektivität der meisten Katalysatortypen aber stark temperaturabhängig ist, kann es aufgrund der Temperaturänderungen im Reaktor zu Veränderungen des Reaktionsmechanismus kommen, so daß daher meist ungewünschte Neben- oder Folgereaktionen stattfinden. Zusätzlich können Übertemperaturen zu einer irreversiblen Schädigung des Katalysators führen.

Zur Begrenzung der Temperaturerhöhung im Reaktor ist es bekannt, die Gasphase in hohem Überschuß durch den Reaktor zu führen oder mehrere, hintereinander geschaltete Reaktoren mit Zwischenkühlung zu benutzen. Diese Art der Temperaturführung ist aber für die Hydrierung von Fettsäure-Triglyceriden nicht ausreichend, da sich die unerwünschten Folgereaktionen so nicht ausschließen lassen und die Katalysatoren bei der Überschreitung gewisser Temperaturen durch Rekristallisationsvorgänge und Strukturveränderungen Aktivitätsverlust erleiden können.

Es ist auch bekannt, derartige katalytische Reaktionen in einem isotherm betriebenen Rohrbündelreaktor durchzuführen (Chemie-Technik, 4. Jahrgang (1975), Nr. 12, S. 439-441). Nach dem sogenannten Bayer-Kalthydrier-Prozeß werden katalytische Hydrierungen bei niedrigen Temperaturen nahezu isotherm durchgeführt, so daß der Katalysator keinen Temperaturschwankungen unterworfen ist und die Gefahr der Überhitzung nicht besteht. Bei der in dieser Literaturstelle beschriebenen Reaktion handelt es sich jedoch um eine recht unkritische Hydrierreaktion, d.h. es wird nur ein Wertprodukt bevorzugt und das Reaktionsprodukt unterliegt nicht oder nicht in diesem Maße der Weiterreaktion, so daß notfalls unumgesetzte Anteile im Kreis geführt werden können oder auch eine verlängerte Verweildauer unter Hydrierbedingungen das Produkt nicht schädigt.

Aufgabe der Erfindung ist somit die Schaffung einer Lösung, mit welcher eine direkte katalytische Hydrierung von Triglyceriden zu Fettalkoholen und Propandiol unter großtechnischen Bedingungen mit Ausbeuten an Propandiol von mehr als 80 % des theoretisch möglichen Wertes und mit einem Gehalt an Paraffinen von nicht mehr als 0,5 % des theoretisch möglichen Wertes ermöglicht wird.

Diese Aufgabe wird mit einem Verfahren der eingangs bezeichneten Art erfindungsgemäß dadurch gelöst, daß die Hydrierreaktion in einem über ein Kühl- oder Heizfluid eingestellten Rohrbündelreaktor durchgeführt wird, wobei die flüssige Phase ohne Rückvermischung neben der gasförmigen Phase als Gleichstromrieselphase über Katalysatorschüttungen in den Reaktoreinzelrohren geleitet wird, und daß das entstehende Reaktionsgemisch auf eine Ausbeute an Fettalkoholen von mindestens 99 % der Theorie und an 1,2-Propandiol von mindestens 80 % der Theorie sowie auf einen Paraffingehalt von höchstens 0,5 % der Theorie eingestellt wird, indem die Volumenbelastung des Reaktors zwischen 0,2 und 2,5 mit der Einsatzstoff pro/l Reaktorvolumen und Stunde und die Flächenbelastung jedes Reaktoreinzelrohres zwischen 1,5 und 24 $m^3$ Einsatzstoff pro/ $m^2$ Reaktorquerschnitt und Stunde gewählt wird und die Reaktionsparameter Temperatur und Druck der aktuellen Katalysatoraktivität entsprechend angepaßt werden.

Es wurde überraschend gefunden, daß mit dieser Verfahrensführung die Hydrierreaktion so gesteuert werden kann, daß die Reaktion auf der Stufe der gewünschten Reaktionsprodukte angehalten wird, so daß sich eine Ausbeute an 1,2-Prophandiol von wenigstens 80 % des theoretisch möglichen Wertes und ein Paraffingehalt von höchstens 0,5 % des theoretisch möglichen Wertes einstellt. Diese Reaktionssteuerung wird dadurch erreicht, daß die fluiden Phasen ohne Rückvermischung mit einer definierten Verweilzeit durch die Katalysatorschüttungen in den Reaktoreinzelrohren geführt werden. Dabei werden die Reaktionsparameter Temperatur und Druck entsprechend der jeweiligen Katalysatoraktivität solange aufeinander abgestimmt, bis sich die gewünschten Produktausbeuten einstellen. Die isotherme Temperaturführung im Rohrbündelreaktor gewährleistet, daß nur die gewünschten Reaktionsmechanismen ablaufen.

Nach einer bevorzugten Ausführungsform der Erfindung ist vorgesehen, daß das Reaktionsgemisch auf eine Ausbeute an 1,2-Propandiol größer 80, vorzugsweise größer 90 und insbesondere größer 95 % der Theorie durch Anpassung der Reaktionsparameter Temperatur und Druck eingestellt wird. Dabei werden die Reaktionsparameter Temperatur und Druck solange aufeinander abgestimmt, bis sich die gewünschen Produktausbeuten einstellen.

In Ausgestaltung sieht die Erfindung vor, daß zur Vermeidung von Rückvermischung der Innendurchmesser der Reaktoreinzelrohre zwischen 25 und 200 mm, vorzugsweise 30 und 100 mm, insbesondere 40 bis 70 mm gewählt wird, und die mobilen Phasen in Pfropfstromcharakteristik durch die Katalysatorschüttung geleitet werden. Durch diese Ausgestaltung der Reaktoreinzelrohre wird sichergestellt, daß die fluiden Phasen in Pfropfstromcharackteristik durch die Katalysatorschüttung strömen, so daß keine Rückvermischung auftreten kann, die zu unkontrollierten Reaktionsabläufen führen könnte. Somit wird eine exakte Reaktionsführung zur Erzielung des gewünschten Reaktionsproduktes gewährleistet.

Besonders zweckmäßig ist es, wenn die Volumenbelastung auf Werte zwischen 0,3 und 2,0 l Einsatzstoff pro/l Reaktorvolumen und Stunde eingestellt wird. Dabei wird die Flächenbelastung jedes Reaktoreinzelrohres vorteilhaft auf Werte zwischen 1,5 und 15 $m^3$ Einsatzstoff pro/$m^2$ Reaktorquerschnitt und Stunde eingestellt. Mit diesen speziellen Verfahrensbedingungen ist eine besonders exakte Reaktionsführung möglich.

Eine weitere bevorzugte Ausführungsform der Erfindung sieht vor, daß durch interne Kühlung über einen Wasserstoffüberschuß und/oder durch externe Kühlung über das Kühlfluid die maximale Temperaturerhöhung in der Reaktionszone auf Werte bis maximal 5 °C eingestellt wird. Durch diese sehr genaue Temperaturführung ist sichergestellt, daß keine unerwünschten Folgereaktionen auftreten und der Katalysator nicht thermisch geschädigt wird.

Nach weiteren Ausgestaltungen der Erfindung ist vorgesehen, daß das Verfahren bei Temperaturen zwischen 180 und 250 °C und bei Drücken von 150 bis 280 bar durchgeführt wird. Diese Verfahrensparameter haben sich als besonders günstig erwiesen.

Besonders gute Hydriererergebnisse werden erreicht, wenn als Katalysatoren stückige oder geformte Katalysatoren auf Basis Kupfer-Chromit eingesetzt werden, wie sie in der DE 36 24 812 der Anmelderin beschrieben sind.

Schließlich sieht die Erfindung in zweckmäßiger Ausgestaltung auch vor, daß die Reaktoreinzelrohre über einen Verteiler gleichmäßig mit einer Genauigkeit von 5 % mit flüssiger Phase beaufschlagt werden. Dadurch ist gewährleistet, daß in allen Reaktionsrohren ein einheitlicher Reaktionsablauf stattfindet, so daß ein einheitliches Reaktionsprodukt entsteht.

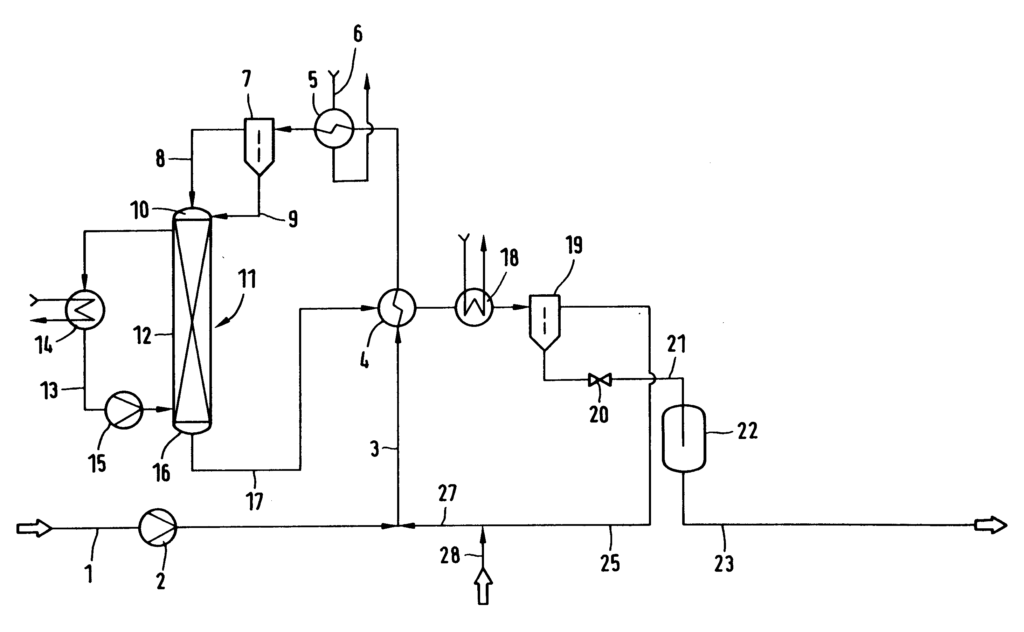

Die Erfindung ist nachstehend anhand der Zeichnung beispielsweise näher erläutert. Diese zeigt in der einzigen Figur ein vereinfachtes Verfahrensfließbild einer Anlage zur Hydrierung von Triglyceriden.

Eine Anlage zur direkten Hydrierung von Fettsäure-Triglyceriden weist eine Leitung 1 mit einer Pumpe 2 auf, mit welcher flüssige Fettsäure-Triglyceride in die Anlage gefördert werden. Die Leitung 1 mündet in eine Leitung 3, die über einen ersten Wärmeaustauscher 4 und einen zweiten Wärmeaustauscher 5, der über eine Leitung 6 mit einem Heizfluid, beispielsweise Hochdruckdampf, beheizt wird, in einen Gas-Flüssig-Abscheider 7. Vom Abscheider 7 geht eine Gasleitung 8 und eine Flüssigleitung 9 aus, welche in den Kopfbereich 10 eines Rohrbündelreaktors 11 geführt sind.

Der vereinfacht dargestellte Rohrbündelreaktor 11 weist in der Reaktionszone 12 eine Vielzahl von parallelen Reaktoreinzelrohren auf, welche mit Katalysatorschüttungen, beispielsweise auf Basis Kupfer-Chromit gefüllt sind. Die Einzelrohre werden über eine Kühlmittelkreislauf 13 im Gegenstrom gekühlt. Dabei ist im Kühlmittelkreislauf 13 ein Wärmeaustauscher 14 zur Kühlung des Kühlfluids und eine Pumpe 15 angeordnet.

Vom Fuß 16 des Rohrbündelreaktors 11 geht eine Produktleitung 17 aus, die zunächst durch den Wärmeaustauscher 4 und anschließend durch einen weiteren Wärmeaustauscher 18 geführt ist. Die Leitung 17 mündet in einen Gas-Flüssig-Abscheider 19. Dabei werden die flüssigen Reaktionsprodukte, insbesondere Fettalkohole und 1,2-Propandiol, aus dem Abscheider 19 über eine Leitung 21 mit Drossel 20 in einen Vorratsbehälter 22 geführt. Das Produkt kann über eine Leitung 23 entnommen werden.

Die aus dem Abscheider 19 austretende, im wesentlichen Wasserstoff aufweisende Gasphase wird über eine Leitung 25 abgeführt. Die Leitung 25 ist in eine Leitung 27 geführt, in die gleichzeitig eine Zuführleitung 28 für Frisch-Wasserstoff mündet. Die Leitung 27 ist im Anschluß an die Einmündungsstelle der Leitung 28 in die Leitung 3 geführt.

Der Verfahrensablauf ist der folgende:

Flüssige Fettsäure-Triglyceride und gasförmiger Wasserstoff werden durch die Leitungen 1 und 28 bzw. 27 in der Leitung 3 zusammengeführt und in den Wärmeaustauschern 4 und 5 auf eine Temperatur oberhalb etwa 160 °C erwärmt. Anschließend werden die beiden Phasen im Abscheider 7 wieder voneinander getrennt und über die Leitungen 8 und 9 am Kopf 10 des Rohrbündelreaktors 11 in den Reaktor 11 eingeleitet. Dabei wird die flüssige Phase, was in der Zeichnung nicht näher dargestellt ist, über einen Verteiler gleichmäßig auf die Einzelrohre des Rohrbündelreaktors 11 verteilt, während sich der gasförmige Wasserstoff ohne besondere Verteileinrichtung automatisch in etwa gleichmäßig auf die Einzelrohre auftrennt.

Die flüssige Phase strömt neben der Gasphase als Rieselphase im Gleichstrom durch die Katalysatorschüttungen in den Einzelrohren. Aufgrund der geometrischen Abmessungen der Reaktoreinzelrohre und einer exakt eingestellten Volumen- und Flächenbelastung der Einzelrohre stellt sich in den Katalysatorrohren eine Pfropfstromcharakteristik ein, so daß keine Rückvermischung auftreten kann. Außerdem wird über eine genaue Temperaturregelung über den Kühlkreislauf 13 bzw. über einen Wasserstoffüberschuß eine exakte Reaktionsführung gewährleistet. Dabei sind die Reaktionsparameter Druck und Temperatur entsprechend der aktuellen Katalysatoraktivität und den eingestellten Strömungsverhältnissen im Reaktor so eingestellt, daß das Hydrierprodukt die gewünschte hohe Ausbeute an Fettalkoholen und 1,2-Propandiol sowie einen geringen Paraffingehalt aufweist.

Das Reaktionsprodukt tritt am Fuß 16 des Reaktors 11 aus den Einzelrohren aus, so daß es nicht mehr mit dem Katalysator in Kontakt kommen kann und somit keine unerwünschten Folgereaktionen ablaufen können, welche die gewünschte Produktzusammensetzung beeinträchtigen könnten. Das Reaktionsprodukt und überschüssiger Sauerstoff werden anschließend über die Leitung 17 im Wärmeaustauscher 4, durch den gleichzeitig neu in die Anlage eingeleites Einsatzprodukt erwärmt wird, und zusätzlich im Wärmeaustauscher 18 abgekühlt, sowie anschließend dem Gas-Flüssig-Abscheider 19 zugeführt. Das flüssige, Fettalkohole und 1,2-Propandiol aufweisende Reaktionsprodukt wird über die Leitung 21 dem Vorratsbehälter 22 zugeführt, aus dem es über die Leitung 23 entnommen werden kann.

Die im Abscheider 19 abgeschiedene Gasphase, welche im wesentlichen Wasserstoff enthält, wird durch die Leitung 25 abgeführt, mit Frisch-Wasserstoff aus der Leitung 28 gemischt und durch die Leitungen 27 und 3 Im Kreislauf in den Rohrbündelreaktor 11 zurückgeführt.

Zum Erhalt des gewünschten Hydrierproduktes, welches eine Ausbeute an Fettalkoholen von mindestens 99 % des theoretisch möglichen Wertes und an 1,2-Propandiol von mindestens 80 % des theoretisch möglichen Wertes sowie einen maximalen Paraffingehalt von 0,5 % des theoretisch möglichen Wertes aufweisen soll, werden die Reaktionsparameter Druck und Temperatur jeweils an die sich im Laufe der Zeit ändernden Katalysatoraktivitäten angepaßt.

Natürlich ist die Erfindung nicht auf das in der Zeichnung dargestellte Ausführungsbeispiel beschränkt. Weitere Ausgestaltungen der Erfindung sind möglich, ohne den Grundgedanken zu verlassen. So kann die isotherme Reaktionsführung im Reaktor auch nur durch Kühlung ohne Wasserstoffüberschuß erfolgen.

**Patentansprüche**

**1.** Verfahren zur katalytischen Hydrierung von flüssigen Fettsäure-Triglyceriden zur gleichzeitigen Gewinnung von Fettalkoholen und $C_3$-Diolen in Gegenwart von gasförmigem Wasserstoff und Hydrierkatalysatoren bei Drücken von 50 bis 300 bar und Temperaturen von 160 bis 250 °C,
dadurch gekennzeichnet,
daß die Hydrierreaktion in einem über ein Kühl- oder Heizfluid isotherm eingestellten Rohrbündelreaktor durchgeführt wird, wobei die flüssige Phase ohne Rückvermischung neben der gasförmigen Phase als Gleichstromrieselphase über Katalysatorschüttungen in den Reaktoreinzelrohren geleitet wird, und daß das entstehende Reaktionsgemisch auf eine Ausbeute an Fettalkoholen von mindestens 99 % der Theorie und an 1,2-Propandiol von mindestens 80 % der Theorie sowie auf einen Paraffingehalt von höchstens 0,5 % der Theorie eingestellt wird, indem die Volumenbelastung des Reaktors zwischen 0,2 und 2,5 l Einsatzstoff pro/l Reaktorvolumen und Stunde und die Flächenbelastung jedes Reaktoreinzelrohres zwischen 1,5 und 24 $m^3$ Einsatzstoff pro/$m^2$ Reaktorquerschnitt und Stunde gewählt wird und die Reaktionsparameter Temperatur und Druck der aktuellen Katalysatoraktivität entsprechend angepaßt werden.

**2.** Verfahren nach Anspruch 1,
dadurch gekennzeichnet,
daß das Reaktionsgemisch auf eine Ausbeute an 1,2-Propandiol größer 80, vorzugsweise größer 90 und insbesondere größer 95 % der Theorie durch Anpassung der Reaktionsparameter Temperatur und Druck eingestellt wird.

**3.** Verfahren nach Anspruch 1 oder 2, dadurch gekennzeichnet, daß zur Vermeidung von Rückvermischung der Innendurchmesser der Reaktoreinzelrohre zwischen 25 und 200 mm, vorzugsweise 30 und 100 mm, insbesondere 40 bis 70 mm gewählt wird, und die mobilen Phasen in Pfropfstromcharakteristik durch die Katalysatorschüttung geleitet werden.

**4.** Verfahren nach Anspruch 1 oder einem der folgenden, dadurch gekennzeichnet, daß die Volumenbelastung auf Werte zwischen 0,3 und 2,0 l Einsatzstoff pro/l Reaktorvolumen und Stunde eingestellt wird.

**5.** Verfahren nach Anspruch 1 oder einem der folgenden, dadurch gekennzeichnet, daß die Flächenbelastung jedes Reaktoreinzelrohres auf Werte zwischen 1,5 und 15 $m^3$ Einsatzstoff pro/$m^2$ Reaktorquerschnitt und Stunde eingestellt wird.

**6.** Verfahren nach Anspruch 1 oder einem der folgenden, dadurch gekennzeichnet, daß durch interne Kühlung über einen Wasserstoffüberschuß und/oder durch externe Kühlung über das Kühlfluid die maximale Temperaturerhöhung in der Reaktionszone auf Werte bis maximal 5 ° C eingestellt wird.

**7.** Verfahren nach Anspruch 1 oder einem der folgenden, dadurch gekennzeichnet, daß es bei Temperaturen zwischen 180 und 250 ° C durchgeführt wird.

**8.** Verfahren nach Anspruch 1 oder einem der folgenden, dadurch gekennzeichnet, daß es bei Drücken von 150 bis 280 bar durchgeführt wird.

**9.** Verfahren nach Anspruch 1 oder einem der folgenden, dadurch gekennzeichnet, daß als Katalysatoren stückige oder geformte Katalysatoren auf Basis Kupfer-Chromit eingesetzt werden.

**10.** Verfahren nach Anspruch 1 oder einem der folgenden, dadurch gekennzeichnet, daß die Reaktoreinzelrohre über einen Verteiler gleichmäßig mit einer Genauigkeit von 5 % mit flüssiger Phase beaufschlagt werden.

## Claims

**1.** A process for the catalytic hydrogenation of liquid fatty acid triglycerides for simultaneously obtaining fatty alcohols and $C_3$ diols in the presence of gaseous hydrogen and hydrogenation catalysts under pressures of 50 to 300 bar and at temperatures of 160 to 250 ° C, characterized in that the hydrogenation reaction is carried out in a tube-bundle reactor isothermally conditioned by a cooling or heating fluid, the liquid phase being passed through catalyst beds in the individual reactor tubes as a co-current trickle phase along with the gas phase without any back-mixing and in that the reaction mixture formed is adjusted to a yield of fatty alcohols of at least 99% of the theoretical and a yield of 1,2-propanediol of at least 80% of the theoretical and to a paraffin content of at most 0.5% of the theoretical by selecting the volume loading of the reactor between 0.2 and 2.5 l starting material per litre reactor volume per hour and the surface loading of each individual reactor tube between 1.5 and 24 $m^3$ starting material per $m^2$ reactor cross-section per hour and adapting the reaction temperature and pressure to the actual catalyst activity.

**2.** A process as claimed in claim 1, characterized in that the reaction mixture is adjusted to a yield of 1,2-propane diol of greater than 80, preferably greater than 90 and more preferably greater than 95% of the theoretical by adaptation of the reaction temperature and pressure.

**3.** A process as claimed in claim 1 or 2, characterized in that, to avoid back-mixing, the internal diameter of the individual reactor tubes is selected between 25 and 200 mm, preferably between 30 and 100 mm and more preferably between 40 and 70 mm and the mobile phases are passed through the catalyst bed in plug flow.

**4.** A process as claimed in claim 1 or any of the following claims, characterized in that the volume loading is adjusted to values of 0.3 to 2.0 l starting material per l reactor volume per hour.

**5.** A process as claimed in claim 1 or any of the following claims, characterized in that the surface loading of each individual reactor tube is adjusted to values of 1.5 to 15 $m^3$ starting material per $m^2$ reactor cross-section per hour.

6. A process as claimed in claim 1 or any of the following claims, characterized in that the maximum temperature increase in the reaction zone is adjusted to at most 5 ° C by internal cooling through an excess of hydrogen and/or by external cooling through the cooling fluid.

7. A process as claimed in claim 1 or any of the following claims, characterized in that it is carried out at temperatures of 180 to 250 ° C.

8. A process as claimed in claim 1 or any of the following claims, characterized in that it is carried out under pressures of 150 to 280 bar.

9. A process as claimed in claim 1 or any of the following claims, characterized in that pelleted or shaped catalysts based on copper chromite are used as the catalysts.

10. A process as claimed in claim 1 or any of the following claims, characterized in that liquid phase is uniformly delivered to the individual catalyst tubes through a distributor with an accuracy of 5%.

**Revendications**

1. Procédé d'hydrogénation catalytique de triglycérides d'acides gras pour obtenir simultanément des alcools gras et des diols en $C_3$ en présence d'hydrogène gazeux et de catalyseurs d'hydrogénation à des pressions comprises entre 50 et 300 bars et des températures comprises entre 160 et 250 ° C, caractérisé en ce que la réaction d'hydrogénation est réalisée dans un réacteur à faisceau tubulaire piloté isothermiquement par un fluide de refroidissement ou de chauffage, on introduit la phase liquide sans risque de remélange à côté de la phase gazeuse comme phase continue de ruissellement sur l'empilage de catalyseur dans les tubes individuels de réacteur et on règle le mélange réactionnel formé à un rendement en alcools gras d'au moins 99 % de la théorie et en 1,2-propane-diol d'au moins 80 % de la théorie ainsi qu'à une teneur en paraffine d'au plus 0,5 % de la théorie en choisissant la charge volumique du réacteur entre 0,2 et 2,5 l de substance mise en oeuvre par l de volume de réacteur et par heure et la charge superficielle de chaque tube individuel de réacteur entre 1,5 et 24 $m^3$ de substance mise en oeuvre par $m^2$ de section de réacteur et par heure et on adapte les paramètres de réaction température et pression à l'activité réelle de catalyseur.

2. Procédé selon la revendication 1, caractérisé en ce qu'on ajuste le mélange réactionnel à un rendement en 1,2-propane-diol supérieur à 80, de préférence supérieur à 90 et notamment supérieur à 95 % de la théorie par adaptation des paramètres de réaction température et pression.

3. Procédé selon la revendication 1 ou 2, caractérisé en ce que pour éviter le remélange, on choisit le diamètre intérieur des tubes individuels de réacteur entre 25 et 200 mm, de préférence entre 30 et 100 mm, notamment 40 à 70 mm et on introduit les phases mobiles en écoulement à chicanes à travers l'empilage de catalyseur.

4. Procédé selon la revendication 1 ou l'une des suivantes, caractérisé en ce qu'on règle la charge volumique à des valeurs comprises entre 0,3 et 2,0 l de substance mise en oeuvre par litre en volume de réacteur et par heure.

5. Procédé selon la revendication 1 ou l'une des suivantes, caractérisé en ce que la charge superficielle de chaque tube individuel de réacteur est réglée à des valeurs comprises entre 1,5 et 15 $m^3$ de substance mise en oeuvre par $m^2$ de section de réacteur et par heure.

6. Procédé selon la revendication 1 ou l'une des suivantes, caractérisé en ce que par refroidissement interne par un excès d'hydrogène et/ou par refroidissement externe par le fluide de refroidissement, on limite l'augmentation maximale de température dans la zone réactionnelle à des valeurs de 5 ° C au maximum.

7. Procédé selon la revendication 1 ou l'une des suivantes, caractérisé en ce qu'on le réalise à des températures comprises entre 180 et 250 ° C.

8. Procédé selon la revendication 1 ou l'une des suivantes, caractérisé en ce qu'on le réalise à des pressions comprises entre 150 et 280 bars.

9. Procédé selon la revendication 1 ou l'une des suivantes, caractérisé en ce qu'on utilise des catalyseurs moulés ou en morceaux à base de chromite de cuivre.

10. Procédé selon la revendication 1 ou l'une des suivantes, caractérisé en ce qu'on charge de façon régulière par un répartiteur, les tubes individuels de réacteur, en phase liquide avec une précision de 5 %.

1
2
3
4
5
6
7
8
9
10
11
12
13
14
15
16
17
18
19
20
21
22
23
25
27
28